(19)

Europäisches
Patentamt
European
Patent Office
Office européen
des brevets

(11) **EP 2 660 595 A1**

(12) **EUROPEAN PATENT APPLICATION**
published in accordance with Art. 153(4) EPC

(43) Date of publication:
**06.11.2013 Bulletin 2013/45**

(21) Application number: **11854102.8**

(22) Date of filing: **26.12.2011**

(51) Int Cl.:
**G01N 33/15** [(2006.01)]

(86) International application number:
**PCT/JP2011/080018**

(87) International publication number:
**WO 2012/090909 (05.07.2012 Gazette 2012/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.12.2010 JP 2010289217**

(71) Applicant: **Astellas Pharma Inc.
Tokyo 103-8411 (JP)**

(72) Inventors:
• **MIZOGUCHI, Ryo
  Tokyo 103-8411 (JP)**
• **GATO, Katsuhiko
  Tokyo 103-8411 (JP)**

(74) Representative: **Gille Hrabal
Patentanwälte
Brucknerstrasse 20
40593 Düsseldorf (DE)**

(54) **METHOD FOR MEASUREMENT OF FAT SOLUBILITY**

(57)  An object of the present invention is to provide a method for rapidly and accurately determining the lipophilicity of a wide variety of substances. The lipophilicity of a wide variety of substances can be accurately and rapidly determined by making lipophilicity determination using multiple solvent systems with varied ratios of hydrophobic and hydrophilic solvents

EP 2 660 595 A1

**Description**

TECHNICAL FIELD

The present invention relates to a technique for determining drug lipophilicity. In particular, the invention relates to a high-throughput drug lipophilicity determination technique.

BACKGROUND ART

In recent years, it has been possible to synthesize a great number of compounds thanks to improved efficiency of combinatorial chemistry and derivative synthesis. Correspondingly, it has been essential for establishing rapid and effective new drug development to rapidly and accurately evaluate the properties of a great number of test compounds at an exploratory stage and strategically select candidate drug compounds. When compounds are explored with an extensive focus on the improvement of pharmacological activity, selected compounds tend to have low solubility. However, in order for a compound to actually function as a pharmaceutical drug, a certain degree of oral absorbability is required, and it is important to accurately identify the characteristics to be possessed by a medicine (i.e., druglikeness), such as lipophilicity, from an early stage of development and strategically run a drug discovery process.

Among the properties of compounds, lipophilicity is an important molecular property that affects various properties such as oral absorbability, membrane permeability, solubility, distribution volume, and metabolic stability. Exemplary lipophilicity parameters that are widely used include logP (partition coefficient) and logD (distribution coefficient), and a common method for determining these coefficients is a shake-flask method. The shake-flask method is a method whereby a flask is charged with a test substance and two types of solvents and is shaken, and then the amounts of the test substance contained in organic and aqueous phases, respectively, are quantified to thereby determine a distribution coefficient. The method for determining an octanol/water distribution coefficient is typically specified in a JIS standard (JIS Z7260-107; OECD Test Guideline 107). Another example available as a simpler system is a HPLC method. The HPLC method is a method whereby the distribution coefficient of a test substance is indirectly determined making use of a correlation observed between retention time on reversed-phase HPLC and distribution coefficient -- specifically, a substance having a known distribution coefficient has been investigated in advance for a correlation between its retention time and the distribution coefficient and then the distribution coefficient of a test substance is determined on the basis of the resulting correlation.

However, the shake-flask method allows lipophilicity to be evaluated accurately but involves complicated steps and is not suitable for processing of a large number of specimens. The HPLC method, unlike the shake-flask method, enables the processing of a large number of specimens but is not applicable to compounds with low water solubility or those which are reactive with a column support. Further, the HPLC method does not allow for accurate determination of a distribution coefficient in the case where a substance having a known distribution coefficient and a test substance significantly differ from each other in their chemical structure.

Thus, a technique for determining lipophilicity by calculation and not by actual measurement has been developed. The distribution coefficient determined by calculation, which is typically expressed as ClogP, is becoming increasingly more precise in recent years but is often discrepant with the distribution coefficient actually measured by the shake-flask method; therefore, the technique for estimating lipophilicity by calculation is not sufficiently accurate.

As an actual measurement for actual rapid and much determination of the lipophilicity of a test substance, lipophilicity determination methods using multiwell plates are proposed in Patent Document 1, Non-patent Documents 1 and 2, and other documents. Non-patent Document 3 discloses a method in which the lipophilicity of a test substance contained in organic and aqueous phases is determined through quantification with three phase types, i.e., the organic phase alone, the aqueous phase alone, and both phases, and by then adopting one of the determined values as appropriate.

CITATION LIST

PATENT DOCUMENT

Patent Document 1: Japanese Unexamined Patent Application Publication No. 2006-267105

NON-PATENT DOCUMENTS

Non-patent Document 1: Yun W. Alelyunas, et al., Journal of Chromatography A, 1217 (2010), 1950-1955
Non-patent Document 2: Y. Dohta, et al., Analytical Chemistry, vol. 79, no. 21, 8312-8315, 2007
Non-patent Document 3: R. Mizoguchi, et al., "23-P8: Building of the High Throughput LogD Measuring System" (poster session material for the 24th Annual Meeting of the Academy of Pharmaceutical Science and Technology, Japan, May 23, 2009)

## SUMMARY OF INVENTION

### TECHNICAL PROBLEM

As described above, the shake-flask method allows lipophilicity to be evaluated relatively accurately but involves complicated steps. And after distributing a sample, this method requires multiple manual steps including performing a phase separation procedure to thereby recover two phases separately, and performing quantitative analysis by HPLC or the like; thus, the method takes time for determination and is not suitable for simultaneous processing of a large number of specimens.

Under these circumstances, a high-throughput, shake flask-based lipophilicity determination method has been proposed, as described in the above-noted documents, which is automatically performed by reducing the amounts of solvents and using multiwell plates such as 96-well ones. However, as the amounts of solvents are reduced, the precision of lipophilicity measurement is more likely to decrease, so the high-throughput lipophilicity determination has been difficult to perform on lowly lipophilic substances with a logD of smaller than 1 and highly lipophilic substances with a logD of greater than 3.

In light of these circumstances, an object of the present invention is to provide a method for rapidly and accurately determining the lipophilicity of a wide variety of substances.

### SOLUTION TO PROBLEM

In order to solve the above-described problems, the present inventors have conducted an extensive study and, as a result, have found that the lipophilicity of a test substance can be accurately and rapidly determined by making lipophilicity determination using at least two types of solvent systems with varied ratios of hydrophobic and hydrophilic solvents. Thus, the inventors have completed the present invention.

More specifically, the present invention includes, but is not limited to, the following:

1. A method for determining the lipophilicity of a test substance, comprising the steps of:

(1) mixing a test substance in a predetermined amount with a first solvent system comprising a hydrophobic solvent and a hydrophilic solvent at a first ratio,
(2) mixing the test substance in a predetermined amount with a second solvent system comprising a hydrophobic solvent and a hydrophilic solvent which are the same as those of the first solvent system at a second ratio,
(3) determining the test substance contained in either the hydrophobic phase or the hydrophilic phase of the first solvent system, after the first solvent system reaches its distribution equilibrium,
(4) determining the test substance contained in the phase of the second solvent system which is other than that determined in the first solvent system, after the second solvent system reaches its distribution equilibrium, and
(5) computing the lipophilicity of the test substance on the basis of two values obtained in steps (3) and (4).

2. The method as set forth in 1, wherein the first ratio of the hydrophobic solvent and the hydrophilic solvent ranges from 50:50 to 1:99 (by volume).
3. The method as set forth in 1 or 2, wherein the second ratio of the hydrophobic solvent and the hydrophilic solvent ranges from 80:20 to 20:80 (by volume).
4. The method as set forth in any one of 1 to 3, wherein the hydrophobic solvent is 1-octanol and the hydrophilic solvent is a buffer solution.
5. The method as set forth in any one of 1 to 4, wherein steps (1) to (5) are performed automatically.
6. The method as set forth in any one of 1 to 5, wherein the method is for use in the test substance with a measured logD value of -2 to 6.
7. The method as set forth in any one of 1 to 6, wherein the lipophilicity of the test substance is computed on the basis of the following equation:

$$(a \times C_1 \times D + b \times C_1) / X = (c \times C_2 + d \times C_2 / D) / Y \qquad \text{(Equation I)}$$

wherein:

D = distribution ratio,
X = amount of the test substance added to the first solvent system,
Y = amount of the test substance added to the second solvent system,
$C_1$ = concentration of the test substance in the hydrophilic phase of the first solvent system,
$C_2$ = concentration of the test substance in the hydrophobic phase of the second solvent system,
a = amount of the hydrophobic solvent (volume of the hydrophobic phase) in the first solvent system,
b = amount of the hydrophilic solvent (volume of the hydrophilic phase) in the first solvent system,
c = amount of the hydrophobic solvent (volume of the hydrophobic phase) in the second solvent system, and
d = amount of the hydrophilic solvent (volume of the hydrophilic phase) in the second solvent system.

8. A system for determining the lipophilicity of a test substance, comprising: a dispenser for dispensing a hydrophobic solvent and a hydrophilic solvent at a first ratio and a second ratio into containers; a dispenser for dispensing a test substance; dispensers for sampling either a hydrophobic phase or a hydrophilic phase of a first solvent system comprising the hydrophobic solvent and the hydrophilic solvent at the first ratio, and for sampling a phase of a second solvent system comprising the hydrophobic solvent and the hydrophilic solvent at the second ratio, the phase which is other than that sampled in the first solvent system; an analyzer for determining the test substance; and a calculator for computing the lipophilicity of the test substance on the basis of the ratios of the hydrophobic solvent and the hydrophilic solvent, and measurement results for the test substance.

9. The system as set forth in 8, wherein the lipophilicity of the test substance is computed on the basis of the following equation:

$$(a \times C_1 \times D + b \times C_1) / X = (c \times C_2 + d \times C_2 / D) / Y \qquad \text{(Equation I)}$$

Wherein:

D = distribution ratio,
X = amount of the test substance added to the first solvent system,
Y = amount of the test substance added to the second solvent system,
$C_1$ = concentration of the test substance in the hydrophilic phase of the first solvent system,
$C_2$ = concentration of the test substance in the hydrophobic phase of the second solvent system,
a = amount of the hydrophobic solvent (volume of the hydrophobic phase) in the first solvent system,
b = amount of the hydrophilic solvent (volume of the hydrophilic phase) in the first solvent system,
c = amount of the hydrophobic solvent (volume of the hydrophobic phase) in the second solvent system, and
d = amount of the hydrophilic solvent (volume of the hydrophilic phase) in the second solvent system.

ADVANTAGEOUS EFFECTS OF INVENTION

The present invention enables rapid and accurate determination of the lipophilicity of substances.

BRIEF DESCRIPTION OF DRAWINGS

FIG. 1 is a graph comparing the logD values determined according to the present invention (Example) with those values determined by a shake-flask method (Comparative Example).
FIG. 2 is a graph comparing the logD values of 14 commercially available compounds which were determined according to the present invention (Example) with those values of the same compounds which were determined by the shake-flask method (Comparative Example).

DESCRIPTION OF EMBODIMENTS

The present invention is directed to a method for determining the lipophilicity of a test substance, comprising the steps of:

(1) mixing a test substance in a predetermined amount with a first solvent system comprising a hydrophobic solvent and a hydrophilic solvent at a first ratio,
(2) mixing the test substance in a predetermined amount with a second solvent system comprising the hydrophobic solvent and the hydrophilic solvent at a second ratio,
(3) determining the test substance contained in either the hydrophobic phase or the hydrophilic phase of the first solvent system, after the first solvent system reaches its distribution equilibrium,
(4) determining the test substance contained in the phase of the second solvent system which is other than that determined in the first solvent system, after the second solvent system reaches its distribution equilibrium, and
(5) computing the lipophilicity of the test substance on the basis of results obtained in steps (3) and (4).

In the present invention, the substance to be determined for lipophilicity is any type of chemical or biological substance and, for example, can be an organic compound, a protein, a peptide or a nucleic acid. Examples of the substance to be determined for lipophilicity according to the present invention include polycyclic aromatic or aliphatic hydrocarbons, halogen-containing aromatic or aliphatic hydrocarbons, nitrogen- and oxygen-containing aromatic or aliphatic hydrocarbons, and lipophilic vitamins. Among these examples, compounds that are acceptable as active ingredients of pharmaceutical drugs, in particular those with a low molecular weight are preferred, those with a molecular weight of not greater than 1000 are more preferred, and nitrogen- and oxygen-containing aromatic or aliphatic hydrocarbons with a molecular weight of not greater than 1000 are still more preferred.
The substance to be determined for lipophilicity can be solid or liquid and, for example, can also be dissolved in a suitable solvent such as DMSO (dimethyl sulfoxide). The lipophilicity of the substance to be determined for lipophilicity according to the present invention is not particularly limited; the present invention enables determination of various substances ranging from lowly lipophilic substances with a logD of not greater than -1 to highly lipophilic ones with a logD of 5 or greater. In particular, the present invention enables accurate lipophilicity determination of even highly lipophilic substances with a logD of greater than 3 and lowly lipophilic ones with a logD of smaller than 1. One embodiment of the test substance in the case of using the method of the present invention is a compound with a logD of -2 to 6, another embodiment is a compound with a logD of -2 to 5, and still another embodiment is a compound with a logD of -1 to 5.
For the purpose of the present invention, the lipophilicity is a parameter that denotes the hydrophobicity of a substance, and specific examples include the distribution coefficient logD and the partition coefficient logP. In general, when two mutually immiscible hydrophilic and hydrophobic solvents are placed into the same container and a test substance is added thereto, the concentration ratio of the substance distributed in a hydrophilic phase and a hydrophobic phase is constant irrespective of the amount of the test substance. More specifically, when the concentration of the test substance in the hydrophilic phase is represented by $C_w$ and that in the hydrophobic phase is represented by $C_o$, the ratio of these concentrations ($C_o/C_w$) is constant; and the common logarithm of this ratio is referred to as the distribution or partition coefficient. As referred to herein, logP (partition coefficient) is a partition coefficient that is not considered for ionization of a test substance and is often used to evaluate the lipophilicity of electrically neutral substances. On the other hand, logD (distribution coefficient) is a parameter that allows for variations in lipophilicity due to molecular dissociation under different pH environments. In particular, substances having a dissociative functional group are commonly evaluated for lipophilicity using logD. Among other things, the logD determined using a buffer solution adjusted to pH 7.4 is referred to as $logD_{7.4}$ and is widely used for lipophilicity evaluation of various substances.
In the present invention, the lipophilicity of a substance is determined using a hydrophobic solvent and a hydrophilic solvent. In this connection, the hydrophobic and hydrophilic solvents are mutually immiscible or almost immiscible. In the present invention, the hydrophobic solvent is so-called a nonpolar solvent and is not or almost not miscible with a polar solvent such as water. In general, the dielectric constant of a hydrophobic solvent is lower than that of a hydrophilic solvent such as water. Examples of the hydrophobic solvent include organic solvents such as 1-octanol (octan-1-ol) and aliphatic hydrocarbons (cyclohexane, dodecane, hexadecane or halogenated hydrocarbons), and in particular 1-octanol is preferably used as the hydrophobic solvent.
On the other hand, the hydrophilic solvent as referred to in the present invention is so-called a polar solvent, i.e., a solvent having electronically charged molecules, and is not or almost not miscible with a hydrophobic solvent. For example, the hydrophilic solvent can be a buffered aqueous solution showing high buffer capacity in a specified pH range (which is to say a buffer solution and is specifically exemplified by a phosphate buffer solution, an acetate buffer solution, a borate buffer solution, and a 3-N-tris(hydroxymethyl)methylamino-2-hydroxypropanesulfonic acid buffer solution). The aforementioned pH range can be a range from pH 0 to 14; and a buffer solution adjusted such that the pH is about 7.4, in particular a phosphate buffer solution buffered at pH 7.4, is preferably used as a hydrophilic solvent. The concentration of a buffer solution can be appropriately adjusted as long as determination is not affected by various factors such as buffer capacity and salt deposition from mixing with a hydrophobic solvent. For example, the concentration of a phosphate buffer solution buffered at pH 7.4 (in terms of phosphoric acid) preferably ranges from 0.5 to 20 mM, more preferably from 1 to 10 mM, and still more preferably from 5 to 10 mM.

As referred to herein, "ratio of a hydrophobic solvent and a hydrophilic solvent" means a "volume ratio of the hydrophobic and hydrophilic phases formed by mixture of the hydrophobic and hydrophilic solvents"; for example, "first solvent system comprising a hydrophobic solvent and a hydrophilic solvent at a first ratio" means a "first solvent system whose volume ratio of the hydrophobic and hydrophilic phases formed by mixture of the hydrophobic and hydrophilic solvents is the first ratio". Accordingly, in consideration of change in total volume due to mixture of the hydrophobic and hydrophilic solvents, and the time taken until distribution equilibrium is achieved, it is preferred that the hydrophobic and hydrophilic solvents be mixed well in advance to prepare a mixture of mutually saturated solvents and the hydrophobic and hydrophilic phases of the mixture be used as hydrophobic and hydrophilic solvents, respectively.

In the present invention, a test substance in a predetermined amount is mixed with a first solvent system comprising a hydrophobic solvent and a hydrophilic solvent at a first ratio, and the test substance in a predetermined amount is mixed with a second solvent system comprising a hydrophobic solvent and a hydrophilic solvent which are the same as those of the first solvent system at a second ratio. By distributing the test substance in different systems with varied phase ratios of the hydrophobic and hydrophilic solvents in this manner, the lipophilicity of a wide variety of substances ranging from highly to poorly lipophilic ones can be determined accurately. Further, as described below, by appropriately selecting the phase ratios of the hydrophobic and hydrophilic solvents, the hydrophilic phase can be sampled without separating and removing the hydrophobic phase, whereby lipophilicity determination can be made; thus, a complicated phase separation procedure can be avoided. In the present invention, the amounts of the test substance to be mixed with the first and second solvent systems, respectively, can be any amounts as long as they are known; they may be the same or different.

In the present invention, the first and second ratios of the hydrophobic and hydrophilic solvents are not particularly limited as long as these ratios are different. In one embodiment of the present invention, the first ratio (by volume) of the hydrophobic and hydrophilic solvents can range, for example, from 50:50 to 1:99, preferably from 20:80 to 1:99, more preferably from 10:90 to 1:99, and still more preferably from 1:14 to 1:49. Increasing the proportion of the hydrophilic solvent is advantageous for the following reasons: in the case where a relatively highly lipophilic substance is determined, the concentration of the test substance in the hydrophilic phase can be increased to improve determination accuracy; and since the upper hydrophobic phase decreases in thickness, when the lower hydrophilic phase is sampled using a dispenser or the like without separating and removing the upper hydrophobic phase, the upper hydrophobic phase can be prevented from being contaminated. In the case of using a solvent system containing a lower amount of the hydrophobic solvent, it is advantageous to sample the hydrophilic phase.

On the other hand, the proportion of a hydrophobic solvent in the second solvent system can be higher than that of the first solvent system. In a preferred embodiment, the second ratio (by volume) of the hydrophobic and hydrophilic solvents ranges from 90:10 to 1:99, and more preferably from 80:20 to 20:80. In such cases as where a very poorly lipophilic substance is determined, the second ratio (by volume) of the hydrophobic and hydrophilic solvents can be set to range from 99:1 to 50:50, so that the amount of the hydrophobic solvent can be increased. Setting the second ratio (by volume) to range from 80:20 to 20:80, in particular from 55:45 to 45:55, is preferred because this improves the determination accuracy in the vicinity of logD=0.

In the present invention, the total amount of the hydrophobic and hydrophilic solvents is not particularly limited but, in terms of performing a high-throughput analysis, preferably ranges from 50 μL to 10 mL, more preferably from 100 μL to 5 mL, still more preferably from 500 μL to 2 mL, yet more preferably from 500 μL to 1.5 mL, and most preferably from 800 μL to 1.2 mL. In terms of determining samples of the respective phases using a mass spectrometer (MS) or the like, when the logD of a test substance approximately ranges from -2 to 5, it is preferred that the first ratio be set to range from 10:90 to 1:99 (more preferably from 1:14 to 1:49) and the second ratio be set to range from 80:20 to 20:80 (more preferably from 55:45 to 45:55), thereby ensuring that the concentrations of the test substance in the first and second solvent systems upon shaking range from 2.5 to 20 μM.

In the present invention, the test substance contained in either the hydrophobic or hydrophilic phase of the first solvent system is determined after the first solvent system reaches its distribution equilibrium, and the test substance contained in the phase of the second solvent system which is other than that determined in the first solvent system, is determined after the second solvent system reaches its distribution equilibrium. Varying the type of the phase to be determined for the test substance between the first and second solvent systems improves determination accuracy. In a preferred embodiment, the test substance contained in the hydrophilic phase is determined in the first solvent system, and that contained in the hydrophobic phase is determined in the second solvent system.

In a preferred embodiment of the present invention, when a compound with a logD of about -2 to 5 is used as a test substance, it is preferred for performing a high-throughput and high-precision analysis that the first ratio be set to range from 10:90 to 1:99 (more preferably from 1:14 to 1:49) and the second ratio be set to range from 80:20 to 20:80 (more preferably from 55:45 to 45:55), thereby ensuring that the total amount of the hydrophilic and hydrophobic solvents ranges from 500 μL to 2 mL.

In the present invention, it is after distribution equilibrium is achieved that the test substance in the hydrophobic phase and/or the hydrophilic phase is determined. In general, distribution equilibrium is achieved after a certain period of time from the addition of a test substance, but the time to achieve distribution equilibrium may be reduced by stirring or shaking. The time to achieve distribution equilibrium, which depends on the amounts of solvents and other factors, ranges for example from 1 minute to 24 hours, preferably from 10 minutes to 12 hours, and more preferably from 30 minutes to 3 hours.

In the present invention, the solvents and the test substance can be placed into and removed from a container by a known method. More specifically, a known dispenser, sampler or other instrument can be used, or these tasks can be automated. It is also preferable to apply the invention to multiple types of test substances using a multiwell plate or the like, because this enables a high-throughput analysis using small amounts of samples. The method of the present invention can be easily automated using a standard system and thus is particularly suitable for a high-throughput analysis.

In the present invention, the method for determining a test substance is not particularly limited; a known method can be used. Specific examples include, but are not limited to, measurement using an absorbance detector such as a UV detector, and measurement using an instrument that combines liquid chromatography (LC), gas chromatography (GC), capillary electrophoresis (CE), or other technique with a suitable detector. Exemplary detectors for liquid chromatography (LC) include an absorbance detector (e.g., UV detector), a mass spectrometer (MS) and/or a conductometric detector. In some cases, determination by LC/MS is preferred for performing a high-throughput and high-precision analysis.

In the present invention, the test substance in the hydrophilic and hydrophobic phases of multiple solvent systems with different phase ratios is determined, and the lipophilicity of the test substance is computed on the basis of the determination results using Equation I:

$$(a \times C_1 \times D + b \times C_1)\,/\,X = (c \times C_2 + d \times C_2\,/\,D)\,/\,Y \qquad (\text{Equation I})$$

wherein:

D = distribution ratio,
X = amount of the test substance added to the first solvent system,
Y = amount of the test substance added to the second solvent system,
$C_1$ = concentration of the test substance in the hydrophilic phase of the first solvent system,
$C_2$ = concentration of the test substance in the hydrophobic phase of the second solvent system,
a = amount of the hydrophobic solvent (volume of the hydrophobic phase) in the first solvent system,
b = amount of the hydrophilic solvent (volume of the hydrophilic phase) in the first  solvent system,
c = amount of the hydrophobic solvent (volume of the hydrophobic phase) in the second solvent system, and
d = amount of the hydrophilic solvent (volume of the hydrophilic phase) in the second solvent system.

In the equation given above, the concentrations $C_1$ and $C_2$ may be replaced with the values that correlate with them; specific examples include LC or LC/MS peak area values.

In the present invention, the distribution ratio D is calculated using the equation given above and, if necessary, the common logarithm is computed to determine logD or logP.

In another embodiment, the present invention is directed to a system for determining the lipophilicity of a test substance. More specifically, the present invention is directed to a system for determining the lipophilicity of a test substance, comprising: a dispenser for dispensing a hydrophobic solvent and a hydrophilic solvent at a first ratio and a second ratio into containers; a dispenser for dispensing a test substance; a dispenser for sampling the hydrophobic phase or the hydrophilic phase of a first solvent system in the container, which comprises the hydrophobic solvent and the hydrophilic solvent at the first ratio, and diluting the sampled phase with a suitable solvent; a dispenser for sampling a phase of a second solvent system comprising the hydrophobic solvent and the hydrophilic solvent at the second ratio, the phase which is other than that sampled in the first solvent system, and diluting the sampled phase with a suitable solvent; a dispenser for sampling the sample prepared by diluting with the suitable solvent the hydrophobic phase or the hydrophilic phase of the first solvent system in the container which comprises the hydrophobic solvent and the hydrophilic solvent at the first ratio, and for sampling the sample prepared by diluting with the suitable solvent the phase of the second solvent system comprising the hydrophobic solvent and the hydrophilic solvent at the second ratio, the phase which is other than that sampled in the first solvent system; an analyzer for determining the test substance; and a calculator for computing the lipophilicity of the test substance on the basis of the ratios of the hydrophobic solvent and the hydrophilic solvent, and measurement results for the test substance. The system of the present invention may further comprise a thermostatic bath for maintaining the containers at a constant temperature, and preferably further comprises a shaker for shaking the containers and/or a centrifugal separator for phase separation of hydrophobic and hydrophilic phases in order to accelerate the test substance to reach its distribution equilibrium. The respective components constituting the inventive system are preferably automated such that a series of analyses are performed automatically.

In order to perform a high-throughput analysis, it is preferable to use a well plate such as a multiwell plate as a container. As described above, it is desirable that the upper organic phase be reduced in thickness because this phase is hard to contaminate upon sampling of the lower hydrophilic phase; hence, the ratios for the solvent systems and the volume (area) of the container are selected such that the amount of the organic phase per $mm^2$ as unit surface area of the liquid in a well preferably ranges from 0.5 to 2 $\mu$L and more preferably is 1 $\mu$L. In terms of various factors such as a high-throughput analysis and the amount of the test substance used, the well volume preferably ranges from 50 $\mu$L to 10 mL and more preferably ranges from 100 $\mu$L to 5 mL, and the cross-sectional area of a well at liquid level preferably ranges from 10 $mm^2$ to 500 $mm^2$ and more preferably ranges from 20 $mm^2$ to 100 $mm^2$. Preferred materials of containers such as plates include polypropylene and glass.

The present invention will be described below in more detail by way of Example, but the present invention is not limited to this example. Unless otherwise specified, all value ranges recited herein include both endpoints.

EXAMPLES

(Example)

A. Lipophilicity determination according to the present invention

Thirty-eight test compounds were determined for $logD_{7.4}$ according to the present invention. The solution amount per well was set to 1 mL, and 1-octanol was used as a hydrophobic solvent and a phosphate buffer solution buffered at pH 7.4 as a hydrophilic solvent. The amounts of the test substance to be added to the first and second solvent systems, respectively, were adjusted to be equal (X = Y in Equation I). As for the first solvent system, the ratio of 1-octanol to a phosphate buffer solution was set to 5:95 (by volume), and the test substance distributed in the phosphate buffer phase (aqueous phase) was determined by liquid chromatography and mass spectrometry (LC/MS). For the second solvent system, the ratio of 1-octanol to a phosphate buffer solution was set to 50:50 (by volume), and the test substance distributed in the 1-octanol phase (hydrophobic phase) was determined by the same method.

Following the above-mentioned conditions, X = Y, a = 5, b = 95, and c = d = 50 were substituted into Equation I to obtain the following quadratic equation (Equation II). As described below, once the test substance was determined, the value of $C_2/C_1$ could be determined on the basis of the peak area values and dilution ratios. The resulting value was substituted into Equation II to determine the distribution ratio D, and the common logarithm of this ratio was obtained to thereby compute $logD_{7.4}$.

$$5 \times C_1 \times D + 95 \times C_1 = 50 \times C_2 + 50 \times C_2 / D$$

$$D^2 + (19 - 10C_2 / C_1) D - 10C_2 / C_1 = 0 \quad \text{(Equation II)}$$

Determination was performed using a 96 deep well plate (Greiner; model No. 780270; top inside well dimensions, L 8.2 mm $\times$ H 8.2 mm; bottom inside well dimensions, L 7.37 mm $\times$ H 7.37 mm; well depth, 41 mm; volume, 2 mL; material, polypropylene). Dispensing, shaking, dilution and other steps were automated by a dispensing robot (Hamilton; MicroLabSTARlet), and transfer of plates between systems and some other tasks were performed manually. The specific test procedure is as follows.

1. Preparation of solutions

(1) Preparation of a phosphate buffer solution (pH 7.4)

Dulbecco's reagent (for 1 L of Sigma's PBS solution; containing 8 g of sodium chloride, 1.15 g of anhydrous sodium monohydrogen phosphate, 0.2 g of potassium chloride, and 0.2 g of anhydrous potassium dihydrogen phosphate) was diluted to give a volume of 1 L, stirring was performed, and insoluble matters were filtered off. The pH of the solution was measured and adjusted to pH 7.4 with 1N HCl.

(2) Preparation of saturated 1-octanol and a saturated phosphate buffer solution

A separating funnel was charged with a sufficient amount of each of 1-octanol (Kanto Chemical; for distribution coefficient determination) and the phosphate buffer solution prepared in (1), they were mixed well, and the mixture was allowed to stand overnight at room temperature. The upper and lower phases were recovered separately to obtain 1-octanol saturated with the phosphate buffer solution (saturated 1-octanol) and the phosphate buffer solution saturated with 1-octanol (saturated phosphate buffer solution). The resulting solutions were stored at room temperature.

(3) DMSO solution of a test compound

A test compound was added to DMSO to prepare a 10 mM solution.

2. Distribution of a test compound

(1) Ten microliters of the 10 mM test compound solution in DMSO and then 490 $\mu$L of saturated 1-octanol were dispensed into a 96 deep well plate. Stirring by suction and ejection was repeated 10 times to prepare a standard octanol solution of the test compound.

(2) Fifty microliters of the standard octanol solution was dispensed into each of two wells; thereafter, 950 $\mu$L of the saturated phosphate buffer solution was dispensed into the first well (hydrophobic solvent:hydrophilic solvent=5:95), and 500 $\mu$L of the saturated phosphate buffer solution and 450 $\mu$L of saturated 1-octanol were dispensed into the second well (hydrophobic solvent:hydrophilic solvent=50:50).

(3) A pressure curable sealant was applied to the multiwell plate, and then the plate was shaken on a shaker at 25°C for 3 hours. After the shaking, centrifugation was performed at 3000 rpm for 5 minutes.

(4) The lower phase (aqueous phase) was drawn from the first well and diluted 10-fold with methanol to thereby prepare a sample. From the second well, the upper phase (1-octanol phase) was drawn and diluted 10-fold and 50-fold with methanol to thereby prepare two additional samples. Thus, a total of 4 different types of samples were provided: the lower phase in the first well (not diluted), the 10-fold diluted sample of the same lower phase, the 10-fold diluted sample of the upper phase in the second well, and the 50-fold diluted sample of the same upper phase, and the test compound was determined by LC/MS using these samples.

3. Determination of a test compound

With these 4 samples placed on an LC/MS auto sampler, determination was performed automatically.

(1) A 2 L volumetric flask was charged with an appropriate amount of ultrapure water (prepared using Millipore's ultrapure water purification system), and then 2 mL of formic acid (Kanto Chemical) was added dropwise using a one-mark pipette. Thereafter, the flask was filled up to its calibration mark with ultrapure water, and the solution was mixed by inversion. The mixture was transferred to a mobile phase bottle and degassed by ultrasonication for 10 minutes, whereby an aqueous solution of 0.1% formic acid was prepared.

(2) A 1 L volumetric flask was charged with an appropriate amount of acetonitrile (Wako Pure Chemical), and then 1 mL of formic acid (Kanto Chemical) was added dropwise using a one-mark pipette. Thereafter, the flask was filled up to its calibration mark with acetonitrile, and the solution was mixed by inversion. The mixture was transferred to a mobile phase bottle and degassed by ultrasonication for 10 minutes to prepare an acetonitrile solution of 0.1% formic acid.

(3) The aqueous solution of 0.1% formic acid (solution A) and the acetonitrile solution of 0.1% formic acid (solution B) were used as a HPLC mobile phase. The gradient conditions were as follows: 0-0.3 min., 10% solution B; 0.3-1.5 min., 10-95% solution B, 1.5-2.25 min., 95% solution B; and 2.25-3.0 min., 10% solution B. The flow rate was set to 0.6 mL/min.

(4) HPLC was performed using Waters Acquity with a binary pump. The column stationary phase used was a reversed-phase column having octadecyl groups (Acquity BEH C18, 1.7 $\mu$m, 2.0 $\times$ 30 mm). The column temperature was set to 50°C.

(5) MS was performed using Waters ZQ or SQD. Data was acquired with the detection mode being set to ESI$^+$ and the type of ion to be detected being set to [M+H]$^+$. The Cone Voltage was set to 25 V. The Cone Gas used was nitrogen gas. The Flow was set to 50L/h and the Desolvation Gas Flow to 750 L/h. The Source Temp was set to 130°C and the Desolvation Gas Temp to 350°C. The Capillary Voltage was set to 3.5 kV.

(6) LogD was computed by the following procedure. Among the lower phase sample of the first well (not diluted), the 10-fold diluted sample of the same lower phase, the 10-fold diluted sample of the upper phase in the second well, and the 50-fold diluted sample of the same upper phase, those samples whose peak area value obtained by LC/MS was higher (i.e., those with a lower dilution ratio) were selected for both phases. The concentration ratio $C_2/C_1$ was computed on the basis of their peak area values and dilution ratios, and the resulting ratio was substituted into Equation II to compute logD automatically. However, in the case where any sample with a lower dilution ratio had a significantly large peak area value, which indicated that the quantitativity of LC/MS was in a lower range (i.e., in the case where any peak area value was 750000 or greater under the conditions of this study), then the samples with a greater dilution ratio were selected and their peak area values were used for computing LogD.

(Comparative Example)

B. Lipophilicity determination by a shake-flask method

Lipophilicity determination was made by a shake-flask method using the following procedure.

1. Preparation of solutions

Respective solutions were prepared by the same procedure as in A.

2. Distribution of a test compound

An appropriate amount (about half a microspatula) of a sample was weighed out and placed into a 10 mL screw cap centrifuge tube. And about 6 mL of the saturated phosphate buffer solution was added in the case of LogD < 0 as determined by a physicochemical property prediction software typified by ACD/Labs (Fujitsu), or about 6 mL of saturated 1-octanol was added in the case of LogD >= 0. Ultrasonication was performed for several  tens of seconds to disperse the test compound, whereby a mixed solution was obtained.
The mixed solution was passed through an Anotop syringe filter, and then about 5 mL of the filtrate was charged into a new 10 mL screw cap centrifuge tube and stirred by inversion 2 or 3 times.
To the screw cap centrifuge tube, the saturated phosphate buffer solution was added in the case of LogD >= 0 as determined by the physicochemical property prediction software, or saturated 1-octanol was added in the case of LogD < 0, so that the centrifuge tube was filled up to the mark of 10 mL.
The screw cap centrifuge tube was vigorously shaken for 10 minutes, and then centrifugation was performed at 3000 rpm for 10 minutes.
One milliliter of the upper phase (1-octanol phase) was weighed out and diluted with methanol to 10 mL. Further samples were taken and diluted with methanol. Thus, 4 different samples, each diluted 10-fold, 100-fold, 1000-fold and 10000-fold, were prepared.
Next, the remaining upper phase was removed completely, and then 1 mL samples of the lower phase (aqueous phase) were taken and diluted with methanol, whereby 4 different samples, each diluted 1-fold (not diluted), 10-fold, 100-fold and 1000-fold, were prepared.

3. Determination of respective phase samples

With these 8 samples placed on an LC/MS auto sampler, determination was performed automatically.

(1) About 1.5 g of ammonium acetate (Wako Pure Chemical) was weighed out into a 2 L volumetric flask, which was filled up with ultrapure water (prepared using Millipore's ultrapure water purification system) so as to give a concentration of 10 mM, and the solution was mixed by inversion. The mixture was transferred to a mobile phase bottle and degassed by ultrasonication for 10 minutes, whereby an aqueous solution of 10 mM ammonium acetate was prepared.
(2) About 1.5 g of ammonium acetate (Wako Pure Chemical) was weighed out into a 2 L volumetric flask, which was filled up with methanol (Wako Pure Chemical) so as  to give a volume of 10 mM, and the solution was mixed by inversion. The mixture was transferred to a mobile phase bottle and degassed by ultrasonication for 10 minutes, whereby a acetonitrile solution of 10 mM ammonium acetate was prepared.
(3) The aqueous solution of 10 mM ammonium acetate (solution A) and the methanol solution of 10 mM ammonium acetate (solution B) were used as a HPLC mobile phase. The gradient conditions were as follows: solution B concentration, 10-50% (0-0.5 min.); 50-100% (0.5-5 min.); and 100% (5-7 min.). The flow rate was set to 0.3 mL/min.
(4) HPLC was performed using Agilent LC1100 with a binary pump. The column stationary phase used was a reversed-phase column having octadecyl groups (Imtakt Cadenza CD-C18; 2.0 ID $\times$ 50 mm). The column temperature was set to 40°C.

(5) MS was performed using Agilent 1946D. The Drying Gas Flow was set to 12.0 L/min., the Drying Gas Temp to 350°C, and the Capillary Voltage to 3000 V. Data was acquired with the detection mode being set to $ESI^+$ and the type of ion to be detected being optimized for each sample. Typical types of ions were [M+H]+ and [M-H]+. The fragmentor voltage was optimized for each sample in the range of 40-200 V, and was typically set to 80 V and 100 V.

(6) LogD was computed by the following procedure. The samples whose peak area obtained by LC/MS was larger (i.e., those with a lower dilution ratio) were selected for both phases, and their peak areas were used for computing logD (1-octanol phase area × dilution factor / aqueous phase area × dilution factor = distribution ratio; the common logarithm of the resulting distribution ratio was obtained to thereby compute logD). However, in the case where any sample with a lower dilution ratio had a significantly large peak area, which indicated that the quantitativity of LC/MS was in a lower range, then the samples with a greater dilution ratio were selected and their peak areas were used for computing LogD.

C. Determination results

Determination was made of not only 14 compounds commercially available but also 24 compounds shown in Table 1 as Compounds 1-24. Table 1 also shows their determined logD values. By performing the above-described determination procedure using 96 well plates, the $logD_{7.4}$ values of these 38 compounds could be determined in about 13 hours, which shows that the present invention enabled an extremely high-throughput analysis.

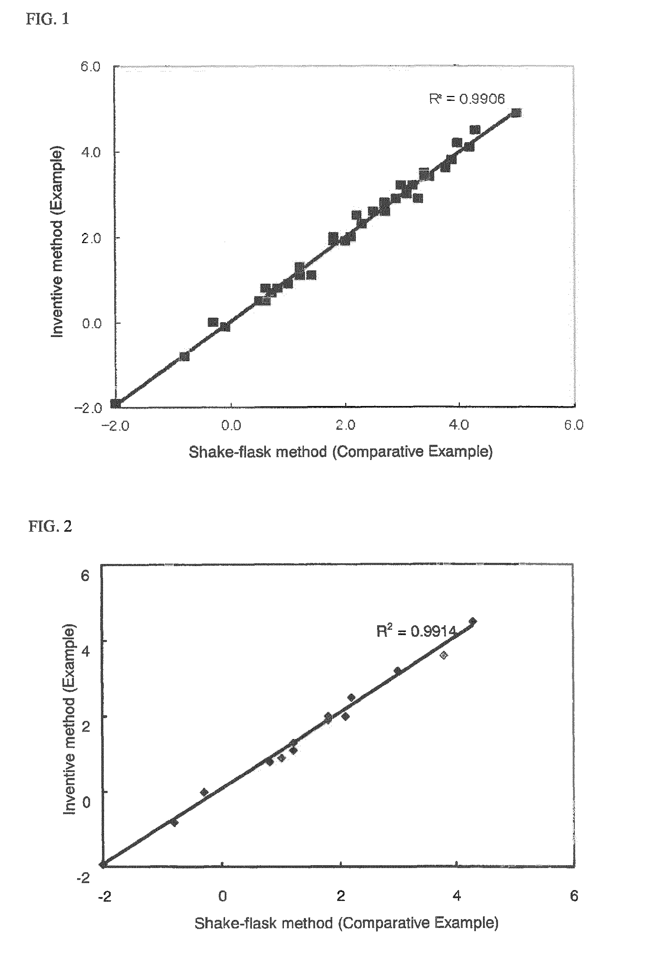

FIG. 1 shows a graph comparing the logD values determined according to the present invention (Example) with those determined by a conventional shake-flask method (Comparative Example). According to FIG. 1, in the range of logD values from -2 to 5, the values determined according to the present invention were almost the same as those determined by the shake-flask method ($R^2$ = 0.9906), which demonstrates that the present invention enables accurate lipophilicity determination. By using two solvent systems with different ratios of hydrophobic and hydrophilic solvents and determining a test substance contained in each of the lower phase of one solvent system and the upper phase of the other system, the hydrophobic phases could be prevented from being contaminated upon sampling and, at the same time, the concentration of the test substance contained in each of the hydrophilic phases could be improved; as a result, lipophilicity determination could be made with high precision.

[Table 1]

| Compound | LogD | | |
|---|---|---|---|
| | Literature value | Shake-flask method (Comparative Example) | Inventive determination method (Example) |
| Quinidine | 1.9 | 2.1 | 2.0 |
| Indomethacin | 0.9 | 1.2 | 1.1 |
| Perphenazine | -- | 3.8 | 3.6 |
| Imipramine | 2.4 | 2.2 | 2.5 |
| Labetalol | 1.1 | 1.0 | 0.9 |
| Probenecid | -- | -0.3 | 0.0 |
| Prazosin | 1.3 | 1.8 | 1.9 |
| Carbamazepine | 1.5 | 1.8 | 2.0 |
| Pioglitazone | -- | 3.0 | 3.2 |
| Propranolol | 1.5 | 1.2 | 1.3 |
| Atenolol | -1.8 | -2.0 | -1.9 |
| Famotidine | -- | -0.8 | -0.8 |
| Tamsulosin | -- | 0.8 | 0.8 |
| Celecoxib | -- | 4.3 | 4.5 |
| Compound 1 | -- | 3.5 | 3.4 |

(continued)

| Compound | LogD | | |
|---|---|---|---|
| | Literature value | Shake-flask method (Comparative Example) | Inventive determination method (Example) |
| Compound 2 | -- | 3.1 | 3.1 |
| Compound 3 | -- | 3.9 | 3.8 |
| Compound 4 | -- | -0.1 | -0.1 |
| Compound 5 | -- | 2.7 | 2.8 |
| Compound 6 | -- | 4.0 | 4.2 |
| Compound 7 | -- | 3.3 | 2.9 |
| Compound 8 | -- | 3.2 | 3.2 |
| Compound 9 | -- | 2.7 | 2.6 |
| Compound 10 | -- | 3.1 | 3.0 |
| Compound 11 | -- | 2.1 | 2.0 |
| Compound 12 | -- | 2.9 | 2.9 |
| Compound 13 | -- | 2.5 | 2.6 |
| Compound 14 | -- | 0.7 | 0.7 |
| Compound 15 | -- | 0.5 | 0.5 |
| Compound 16 | -- | 1.4 | 1.1 |
| Compound 17 | -- | 2.3 | 2.3 |
| Compound 18 | -- | 0.6 | 0.8 |
| Compound 19 | -- | 3.4 | 3.5 |
| Compound 20 | -- | 3.4 | 3.4 |
| Compound 21 | -- | 4.2 | 4.1 |
| Compound 22 | -- | 2.0 | 1.9 |
| Compound 23 | -- | 0.6 | 0.5 |
| Compound 24 | -- | 5.0 | 4.9 |

INDUSTRIAL APPLICABILITY

The present invention enables rapid and accurate determination of the lipophilicity of substances.

**Claims**

1.    A method for determining the lipophilicity of a test substance, comprising the steps of:

(1) mixing a test substance in a predetermined amount with a first solvent system comprising a hydrophobic solvent and a hydrophilic solvent at a first ratio,
(2) mixing the test substance in the predetermined amount with a second solvent system comprising a hydrophobic solvent and a hydrophilic solvent which are the same as those of the first solvent system at a second ratio,
(3) determining the test substance contained in either the hydrophobic phase or the hydrophilic phase of the first solvent system, after the first solvent system reaches its distribution equilibrium,
(4) determining the test substance contained in the phase of the second solvent system which is other than that determined in the first solvent system, after the second solvent system reaches its distribution equilibrium, and
(5) computing the lipophilicity of the test substance on the basis of two values obtained in steps (3) and (4).

2. The method according to Claim 1, wherein the first ratio of the hydrophobic solvent and the hydrophilic solvent ranges from 50:50 to 1:99 (by volume).

3. The method according to Claim 1 or 2, wherein the second ratio of the hydrophobic solvent and the hydrophilic solvent ranges from 80:20 to 20:80 (by volume).

4. The method according to any one of Claims 1 to 3, wherein the hydrophobic solvent is 1-octanol and the hydrophilic solvent is a buffer solution.

5. The method according to any one of Claims 1 to 4, wherein steps (1) to (5) are performed automatically.

6. The method according to any one of Claims 1 to 5, wherein the method is for use in the test substance with a measured logD value of -2 to 6.

7. The method according to any one of Claims 1 to 6, wherein the lipophilicity of the test substance is computed on the basis of the following equation:

$$(a \times C_1 \times D + b \times C_1) / X = (c \times C_2 + d \times C_2 / D) / Y \qquad \text{(Equation I)}$$

wherein:

D = distribution ratio,
X = amount of the test substance added to the first solvent system,
Y = amount of the test substance added to the second solvent system,
$C_1$ = concentration of the test substance in the hydrophilic phase of the first solvent system,
$C_2$ = concentration of the test substance in the hydrophobic phase of the second solvent system,
a = amount of the hydrophobic solvent (volume of the hydrophobic phase) in the first solvent system,
b = amount of the hydrophilic solvent (volume of the hydrophilic phase) in the first solvent system,
c = amount of the hydrophobic solvent (volume of the hydrophobic phase) in the second solvent system, and
d = amount of the hydrophilic solvent (volume of the hydrophilic phase) in the second solvent system.

8. A system for determining the lipophilicity of a test substance, comprising:

a dispenser for dispensing a hydrophobic solvent and a hydrophilic solvent at a first ratio and a second ratio into containers;
a dispenser for dispensing a test substance;
dispensers for sampling either a hydrophobic solvent phase or a hydrophilic solvent phase of a first solvent system comprising the hydrophobic solvent and the hydrophilic solvent at the first ratio, and for sampling a phase of a second solvent system comprising the hydrophobic solvent and the hydrophilic solvent at the second ratio, the phase which is other than that sampled in the first solvent system;
an analyzer for determining the test substance; and
a calculator for computing the lipophilicity of the test substance on the basis of the ratios of the hydrophobic solvent and the hydrophilic solvent, and measurement results for the test substance.

9. The system according to Claim 8, wherein the lipophilicity of the test substance is computed on the basis of the following equation:

$$(a \times C_1 \times D + b \times C_1) / X = (c \times C_2 + d \times C_2 / D) / Y \qquad \text{(Equation I)}$$

wherein:

D = distribution ratio,
X = amount of the test substance added to the first solvent system,
Y = amount of the test substance added to the second solvent system,
$C_1$ = concentration of the test substance in the hydrophilic phase of the first solvent system,

$C_2$ = concentration of the test substance in the hydrophobic phase of the second solvent system,

a = amount of the hydrophobic solvent (volume of the hydrophobic phase) in the first solvent system,

b = amount of the hydrophilic solvent (volume of the hydrophilic phase) in the first solvent system,

c = amount of the hydrophobic solvent (volume of the hydrophobic phase) in the second solvent system, and

d = amount of the hydrophilic solvent (volume of the hydrophilic phase) in the second solvent system.

FIG. 1

FIG. 2

## INTERNATIONAL SEARCH REPORT

International application No.

PCT/JP2011/080018

A. CLASSIFICATION OF SUBJECT MATTER
*G01N33/15(2006.01)i*

According to International Patent Classification (IPC) or to both national classification and IPC

B. FIELDS SEARCHED

Minimum documentation searched (classification system followed by classification symbols)
G01N33/15

Documentation searched other than minimum documentation to the extent that such documents are included in the fields searched
Jitsuyo Shinan Koho          1922-1996    Jitsuyo Shinan Toroku Koho   1996-2012
Kokai Jitsuyo Shinan Koho    1971-2012    Toroku Jitsuyo Shinan Koho   1994-2012

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | Ryo MIZOGUCHI, Katsuhiko GATO, Masamichi YUDA, Keitaro MORI, Toshio TERAMURA, "High Throughput LogD Sokutei System no Kochiku", Journal of Pharmaceutical Science and Technology, Japan, 30 April 2009 (30.04.2009), vol.69, page 211 | 1-9 |
| A | JP 2006-267105 A  (F. Hoffmann-LA Roche AG.), 05 October 2006 (05.10.2006), entire text; all drawings & US 2006/0211121 A1     & EP 1705474 A1 & DE 602006013899 D       & CA 2540222 A & CN 1837818 A           & SG 126091 A & AT 466270 T            & ES 2341122 T | 1-9 |

☒  Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 February, 2012 (06.02.12) | 21 February, 2012 (21.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/080018 |

C (Continuation).   DOCUMENTS CONSIDERED TO BE RELEVANT

| Category* | Citation of document, with indication, where appropriate, of the relevant passages | Relevant to claim No. |
|---|---|---|
| A | STOPHER D, MCCLEAN S, An improved method for the determination of distribution coefficients., J Pharm Pharmacol, 1990.02, Vol.42, No.2, Page.144 | 1-9 |
| A | Alelyunas YW, Pelosi-Kilby L, Turcotte P, Kary MB, Spreen RC, A high throughput dried DMSO LogD lipophilicity measurement based on 96-well shake-flask and atmospheric pressure photoionization masss pectrometry detection., J Chromatogr A, 2010.03, Vol.1217, No.12, Page. 1950-1955 | 1-9 |
| A | JP 11-344485 A  (Eisai Co., Ltd.), 14 December 1999 (14.12.1999), entire text (Family: none) | 1-9 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006267105 A

**Non-patent literature cited in the description**

- **YUN W. ALELYUNAS et al.** *Journal of Chromatography A,* 2010, vol. 1217, 1950-1955
- **Y. DOHTA et al.** *Analytical Chemistry,* 2007, vol. 79 (21), 8312-8315
- **R. MIZOGUCHI et al.** 23-P8: Building of the High Throughput LogD Measuring System. *24th Annual Meeting of the Academy of Pharmaceutical Science and Technology, Japan,* 23 May 2009